(19) Europäisches Patentamt
European Patent Office
Office européen des brevets

(11) EP 2 067 485 A1

(12) EUROPEAN PATENT APPLICATION

(43) Date of publication:
10.06.2009 Bulletin 2009/24

(21) Application number: 07122621.1

(22) Date of filing: 07.12.2007

(51) Int Cl.:
A61K 41/00 (2006.01) A61K 49/00 (2006.01)
A61K 49/18 (2006.01) A61K 9/00 (2006.01)
A61K 9/127 (2006.01)

(84) Designated Contracting States:
AT BE BG CH CY CZ DE DK EE ES FI FR GB GR HU IE IS IT LI LT LU LV MC MT NL PL PT RO SE SI SK TR
Designated Extension States:
AL BA HR MK RS

(71) Applicant: Koninklijke Philips Electronics N.V.
5621 BA Eindhoven (NL)

(72) Inventor: The designation of the inventor has not yet been filed

(74) Representative: Van Velzen, Maaike Mathilde
Philips
Intellectual Property & Standards
P.O. Box 220
5600 AE Eindhoven (NL)

# (54) Drug carrier providing MRI contrast enhancement

(57) Described are drug carriers useful in magnetic resonance imaging (MRI)-guided drug release. The carrier comprises a thermosensitive material, e.g. liposomes comprising a thermosensitive shell, so as to enable drug release by the local application of heat. The drug carriers are useful in MRI-guided drug release through activity as a contrast enhancement agent for MRI based on the principle of Chemical Exchange-dependent saturation transfer (CEST).

EP 2 067 485 A1

## Description

FIELD OF THE INVENTION

The invention relates to the Magnetic Resonance Imaging (MRI) monitored or guided delivery of bio-active agents such as therapeutic or diagnostic agents (hereinafter referred to as "drugs"). More particularly, the invention relates to drug carriers to be used as MRI contrast agents in such monitored or guided delivery.

BACKGROUND OF THE INVENTION

Many diseases that are mostly localized in a certain tissue are treated with systemically administered drugs. A well-known example of standard cancer therapy is a systemic chemotherapy coming along with significant side effects for the patient due to undesired biodistribution and toxicity. The therapeutic window of these drugs is usually defined by the minimal required therapeutic concentration in the diseased tissue on the one hand, and the toxic effects in non-targeted organs, e.g. liver, spleen, on the other. Localized treatment by, for example, local release of cytostatics from nanocarriers promises a more efficient treatment and a larger therapeutic window compared to standard therapeutics. Localized drug delivery is also important if other therapeutic options such as surgery are too risky as is often the case for liver cancers. Localized drug delivery can also become the preferred treatment option for many indications in cardiovascular disease (CVD), such as atherosclerosis in the coronary arteries.

Magnetic Resonance Imaging is an important diagnostic technique that is commonly used in hospitals for the diagnosis of disease. MRI allows for the non-invasive imaging of soft tissue with a superb spatial resolution.

As a useful extension of its diagnostic use, MRI is also proposed for the monitoring of the delivery of bio-active agents such as therapeutic or diagnostic agents. I.e., MRI can not only be used for treatment planning, but also to control local drug delivery under image guidance.

A reference in this respect is Ponce et al., J Natl Cancer Inst 2007;99: 53 - 63Herein a drug, doxorubicin, is taken up in a temperature-sensitive liposome that is solid at normal body temperature, and melts at a few degrees higher (41-42°C). Thus, drug release can be facilitated by applying heat, as this will result in the opening-up of the liposome, whereupon drug release is no longer determined by diffusion (if any) through the liposomal shell. In order to monitor drug release by MRI, manganese is added to the formulation as an MRI contrast agent.

Almost all current MRI scans are based on the imaging of bulk water molecules, which are present at a very high concentration throughout the whole body in all tissues. If the contrast between different tissues is insufficient to obtain clinical information, MRI contrast agents (CAs), such as low molecular weight complexes of gadolinium, are administered. These paramagnetic complexes reduce the longitudinal ($T_1$) and transverse relaxation times ($T_2$) of the protons of water molecules. Also manganese acts as a $T_1$ contrast agent.

The manganese contrast agent in the aforementioned drug carrier, will act upon its exposure to the bulk water molecules detected by MRI, i.e. it will lead to instantaneous MRI contrast enhancement upon opening up of the liposomal shell above the melt transition temperature of the lipids, after the application of heat.

As described, the MRI used in this drug release process, is in fact used to monitor the actual release, so as to confirm that the thermo-sensitive liposomes actually work. I.e., it merely provides *ex post facto* information.

SUMMARY OF THE INVENTION

It would be advantageous to monitor the fate of the drug carrier as of its administration. Also, rather than confirming, instantaneously, the fact that the drug is released, such monitoring is desired as to determine in advance if, when and where the drug release should take place. Particularly with thermo-sensitive drug carriers, it would be advantageous to localize the drug carrier, and apply the heat that serves to facilitate drug release on the spot where desired, and the moment in time when desired.

In order to better address the aforementioned desires, the invention, in one aspect, presents a drug carrier suitable for localized drug delivery, comprising a Chemical Exchange-dependent Saturation Transfer (CEST) contrast agent for magnetic resonance imaging (MRI).

In another aspect, the invention provides a thermosensitive drug carrier that can be used as a CEST contrast agent in MRI.

In a further aspect, the invention provides a drug carrier comprising a thermosensitive, semipermeable shell enclosing a cavity, wherein the cavity comprises a paramagnetic chemical shift reagent and a pool of proton analytes, and wherein the shell allows diffusion of the proton analytes.

In yet another aspect, the invention provides a carrier for the controlled localized release of a bio-active agent, such as a therapeutic or diagnostic agent, comprising a thermosensitive, semipermeable shell enclosing a cavity, wherein the cavity comprises a paramagnetic chemical shift reagent and a pool of proton analytes, wherein the shell allows

diffusion of the proton analytes, and wherein the shell can be modulated by heat so as to facilitate release of a bio-active agent comprised in the carrier.

DETAILED DESCRIPTION OF THE INVENTION

In a broad sense, the invention can be described with reference to a drug carrier suitable for localized drug delivery, comprising a CEST contrast agent. The suitability of the drug carrier for localized drug delivery can refer to a variety of ways in which a drug carrier loaded with a drug can be triggered to release the drug locally, e.g. by applying a controlled external force or delivering a sufficient amount of energy. This refers, e.g. to thermosensitive drug carriers that can be triggered to locally release a drug by applying local heat. Other methods for localized delivery do not necessarily involve thermosensitive carriers, but carriers that can be triggered to release a drug by a method of activation governed by properties other than thermosensitivity, including but not limited to pH, the presence of a gaseous core and/or layers, sensitive to externally applied ultrasound frequency/wavelength and intensity, and external transcranial energy (e.g. ultrasound) controlled nanomechanical synthetic cells.

CEST MRI

The invention relates to CEST MRI contrast enhancement. This method serves to generate image contrast by utilizing Chemical Exchange-dependent Saturation Transfer (CEST) from selected, magnetically pre-saturated protons to the bulk water molecules determined by MRI
Although the invention relates to the application of any CEST-type contrast enhancement to thermosensitive drug release, it is preferred to make use of more advanced CEST methods as have become available.
CEST in combination with a paramagnetic chemical shift reagent (ParaCEST) is a method, in which the magnetization of a pool of paramagnetically shifted protons of a CEST contrast agent is selectively saturated by the application of radio frequency (RF) radiation. The transfer of this saturation to bulk water molecules by proton exchange leads to a reduced amount of excitable water protons in the environment of the CEST contrast agent. Thus a decrease of the bulk water signal intensity is observed, which can be used to create a (negative) contrast enhancement in MRI images.
An approach to obtain a high CEST efficiency is based on utilizing the large number of water molecules of a solution containing a paramagnetic shift reagent (e.g. Na[Tm(dotma)($H_2O$)]), wherein "$H_4$dotma" stands for α,α',α,"α"'-tetramethyl-1,4,7,19-tetraacetic acid and dotma represents the respective fourfold deprotonated tetraanionic form of the ligand, to provide a pool of protons that are chemically shifted and that, therefore, can selectively be saturated by an RF pulse. If this system is encapsulated in a carrier, e.g. a liposome, the magnetic saturation can be transferred to the bulk water molecules, at the outside of the carriers, which are not chemically shifted (LipoCEST). The amount of magnetization transfer and hence the extent of contrast enhancement is determined by the rate of the diffusion of water through the shell of the carrier, e.g. a phospholipid membrane, as well as by the amount of water within the carrier.
The optimum water exchange rate is directly correlated with the chemical shift difference between the proton pool inside of the carrier and the bulk water outside of the carrier. The paramagnetic shift that is induced on the water molecules inside the liposomes consists of two main contributions: chemical shift resulting from a direct dipolar interaction between the water molecules and the shift reagent ($\delta_{dip}$), and chemical shift caused by a bulk magnetic susceptibility effect ($\delta_{bms}$). The overall paramagnetic shift is the sum of these two contributions:

$$\delta = \delta_{dip} + \delta_{bms} \qquad (1)$$

$\delta_{bms}$ is zero for spherical particles, but it can be significant for anisotropic particles. The aspherical particles experience a force in a magnetic field, which causes them to align with the magnetic field lines. In the case of liposomes, this effect is further increased, if they bear paramagnetic molecules associated with the phospholipid membrane.
A reference on CEST using aspherical liposomes is Terreno,E. et al. Angew. Chem. Int. Ed. 46, 966-968 (2007).

Drug carrier

A drug carrier in the context of the present invention refers to any material in or on which a bio-active agent can be contained so as to be capable of being released in the body of a subject. Reference is made to the drug carrier comprising a CEST contrast agent. This includes the possibility that the drug carrier as such is adapted for use as a CEST contrast agent.
Suitable carriers include microcarriers and, particularly nanocarriers such as liposomes, polymersomes, nanocapsules and other dosage forms of a size or nature commensurate with a use as a CEST contrast agent.

The drug carrier is to be introduced into the body of a person to be subjected to MRI. This will be e.g. by injection in the blood stream, or by other methods to introduce the carrier into body fluid.

A drug is a chemical substance used in the treatment, cure, prevention, or diagnosis of a disease or disorder, or used to otherwise enhance physical or mental well-being. The guided delivery foreseen with the present invention will mostly be useful therapeutic agents (i.e. drugs in a strict sense, intended for therapy or prevention of diseases or disorders), but also for agents that are administered for diagnostic purposes. Although other bio-active agents, i.e. those that are not therapeutic or diagnostic, such as functional food ingredients, will not generally be subjected to guided and/or monitored delivery, such could be done using the present invention if desired.

The most optimal use of the invention is attained in the case of targeted therapeutics, i.e. drugs that are intended for targeted delivery, as such delivery will by nature benefit most from the monitoring made available by the invention. This pertains, e.g., to agents in the treatment of tumors to be delivered on site, to agents in the treatment or prevention of cardiovascular disorders, such as atherosclerosis in the coronary arteries, or to antithrombotic agents (e.g. for locally resolving blood cloths) or agents that require passing the blood-brain barrier such as neuromodulators as can be used in the treatment of neural conditions such as epilepsy, Alzheimer's disease, Parkinson's disease, or stroke. Benefits from the guidance and monitoring of targeted drug delivery are also applicable to targeted diagnostic agents. Similarly as with targeted therapeutics, here too cancer is an area where site-specific delivery can be of importance.

Bio-active agents suitable for use in the present invention include biologically active agents including therapeutic drugs, endogenous molecules, and pharmacologically active agents, including antibodies; nutritional molecules; cosmetic agents; diagnostic agents; and additional contrast agents for imaging. As used herein, an active agent includes pharmacologically acceptable salts of active agents.

The drug carriers of the present invention can comprise either hydrophilic or hydrophobic bioactive agents. A hydrophilic bioactive agent could be encapsulated in the aqueous compartment of the carrier, whereas hydrophobic bioactive agents could be incorporated in hydrophobic domains of the carrier, for instance in the lipid bilayer of liposomes. Nucleic acids, carbohydrates and, in general, proteins and peptides are water soluble or hydrophilic. For instance, bioactive agents which are small molecules, lipids, lipopolysaccharides, polynucleotides and antisense nucleotides (gene therapy agents) are also envisaged. Such biologically active agents which may be incorporated thus include non-peptide, non-protein drugs. It is possible within the scope of the present invention to incorporate drugs of a polymeric nature, but also to incorporate drugs of a relatively small molecular weight of less than 1500 g/mol, or even less than 500 g/mol.

Accordingly, compounds envisaged for use as bioactive agents in the context of the present invention include any compound with therapeutic or prophylactic effects. It can be a compound that affects or participates in tissue growth, cell growth, cell differentiation, a compound that is able to invoke a biological action such as an immune response, or a compound that can play any other role in one or more biological processes. A non-limiting list of examples includes antimicrobial agents (including antibacterial, antiviral agents and anti-fungal agents), anti-viral agents, anti-tumor agents, thrombin inhibitors, antithrombogenic agents, thrombolytic agents, fibrinolytic agents, vasospasm inhibitors, calcium channel blockers, vasodilators, antihypertensive agents, antimicrobial agents, antibiotics, inhibitors of surface glycoprotein receptors, antiplatelet agents, antimitotics, microtubule inhibitors, anti secretory agents, actin inhibitors, remodeling inhibitors, anti metabolites, antiproliferatives (including antiangiogenesis agents), anticancer chemotherapeutic agents, anti-inflammatory steroid or non-steroidal anti-inflammatory agents, immunosuppressive agents, growth hormone antagonists, growth factors, dopamine agonists, radiotherapeutic agents, extracellular matrix components, ACE inhibitors, free radical scavengers, chelators, antioxidants, anti polymerases, and photodynamic therapy agents.

Relatively small peptides may be referred to by the number of amino acids (e.g. di-, tri-, tetrapeptides). A peptide with a relatively small number of amide bonds may also be called an oligopeptide (up to 50 amino acids), whereas a peptide with a relatively high number (more than 50 amino acids) may be called a polypeptide or protein. In addition to being a polymer of amino acid residues, certain proteins may further be characterized by the so called quaternary structure, a conglomerate of a number of polypeptides that are not necessarily chemically linked by amide bonds but are bonded by forces generally known to the skilled professional, such as electrostatic forces and Vanderwaals forces. The term peptides, proteins or mixtures thereof as used herein is to include all above mentioned possibilities.

Usually, the protein and/or peptide are selected on the basis of its biological activity. Depending on the type of polymer chosen, the product obtainable by the present process is highly suitable for controlled release of proteins and peptides. In a particular embodiment, the protein or peptide is a growth factor.

Other examples of peptides or proteins or entities comprising peptides or proteins which may advantageously be contained in the loaded polymer include, but are not limited to, immunogenic peptides or immunogenic proteins, which include, but are not limited to, the following:

Toxins such as diphtheria toxin and tetanus toxin.

Viral surface antigens or parts of viruses such as adenoviruses, Epstein-Barr Virus, Hepatitis A Virus, Hepatitis B Virus, Herpes viruses, HIV-1, HIV-2, HTLV-III, Influenza viruses, Japanese encephalitis virus, Measles virus, Papilloma viruses, Paramyxoviruses, Polio Virus, Rabies, Virus, Rubella Virus, Vaccinia (Smallpox) viruses and Yellow

Fever Virus.

Bacterial surface antigens or parts of bacteria such as *Bordetella pertussis*, *Helicobacter pylori*, *Clostridium tetani, Corynebacterium diphtheria, Escherichia coli, Haemophilus influenza, Klebsiella* species, *Legionella pneumophila, Mycobacterium bovis, Mycobacterium leprae, Mycrobacterium tuberculosis, Neisseria gonorrhoeae, Neisseria meningitidis, Proteus species, Pseudomonas aeruginosa, Salmonella species, Shigella species, Staphylococcus aureus, Streptococcus pyogenes, Vibrio cholera* and *Yersinia pestis* .

Surface antigens of parasites causing disease or portions of parasites such as *Plasmodium vivax* (malaria), *Plasmodium falciparum* (malaria), *Plasmodium ovale* (malaria), *Plasmodium malariae* (malaria), *Leishmania tropica* (leishmaniasis), *Leishmania donovani*), leishmaniasis), *Leishmania branziliensis* (leishmaniasis), *Trypanosoma rhodescense* (sleeping sickness), *Trypanosoma gambiense* (sleeping sickness), *Trypanosoma cruzi* (Chagas' disease), *Schistosoma mansoni* (schistosomiasis), *Schistosomoma haematobium* (schistomiasis), *Schistosoma japonicum* (shichtomiasis), Trichinella spiralis (trichinosis), *Stronglyloides duodenale* (hookworm), *Ancyclostoma duodenale* (hookworm), *Necator americanus* (hookworm), *Wucheria bancrofti* (filariasis), *Brugia malaya* (filariasis), *Loa loa* (filariasis), *Dipetalonema perstaris* (filariasis), *Dracuncula medinensis* (filariasis), and *Onchocerca volvulus* (filariasis).

Immunoglobulins such as IgG, IgA, IgM, Antirabies immunoglobulin, and Antivaccinia immunoglobulin.

Antitoxin such as Botulinum antitoxin, diphtheria antitoxin, gas gangrene antitoxin, tetanus antitoxin.

Antigens which elicit an immune response against foot and mouth disease.

Hormones and growth factors such as follicle stimulating hormone, prolactin, angiogenin, epidermal growth factor, calcitonin, erythropoietin, thyrotropic releasing hormone, insulin, growth hormones, insulin-like growth factors 1 and 2, skeletal growth factor, human chorionic gonadotropin, luteinizing hormone, nerve growth factor, adrenocorticotropic hormone (ACTH), luteinizing hormone releasing hormone (LHRH), parathyroid hormone (PTH), thyrotropin releasing hormone (TRH), vasopressin, cholecystokinin, and corticotropin releasing hormone; cytokines, such as interferons, interleukins, colony stimulating factors, and tumor necrosis factors: fibrinolytic enzymes, such as urokinase, kidney plasminogen activator; and clotting factors, such as Protein C, Factor VIII, Factor IX, Factor VII and Antithrombin III.

Examples of other proteins or peptides are albumin, atrial natriuretic factor, renin, superoxide dismutase, alpha 1 -antitrypsin, lung surfactant proteins, bacitracin, bestatin, cydosporine, delta sleep-inducing peptide (DSIP), endorphins, glucagon, gramicidin, melanocyte inhibiting factors, neurotensin, oxytocin, somostatin, terprotide, serum thymide factor, thymosin, DDAVP, dermorphin, Met-enkephalin, peptidoglycan, satietin, thymopentin, fibrin degradation product, des-enkephalin- alpha -endorphin, gonadotropin releasing hormone, leuprolide, alpha -MSH and metkephamid.

Anti-tumor agents such as altretamin, fluorouracil, amsacrin, hydroxycarbamide, asparaginase, ifosfamid, bleomycin, lomustin, busulfan, melphalan, chlorambucil, mercaptopurin, chlormethin, methotrexate, cisplatin, mitomycin, cyclophosphamide, procarbazin, cytarabin, teniposid, dacarbazin, thiotepa, dactinomycin, tioguanin, daunorubicin, treosulphan, doxorubicin, tiophosphamide, estramucin, vinblastine, etoglucide, vincristine, etoposid, vindesin and paclitaxel.

Antimicrobial agents comprising:

Antibiotics such as ampicillin, nafcillin, amoxicillin, oxacillin, azlocillin, penicillin G, carbenicillin, penicillin V, dicloxacillin, phenethicillin, floxacillin, piperacillin, mecillinam, sulbenicillin, methicillin, ticarcillin, mezlocillin , Cephalosporins: cefaclor, cephalothin, cefadroxil, cephapirin, cefamandole, cephradine, cefatrizine, cefsulodine, cefazolin, ceftazidim, ceforanide, ceftriaxon, cefoxitin, cefuroxime, cephacetrile, latamoxef, and cephalexin. Aminoglycosides such as amikacin, neomycin, dibekacyn, kanamycin, gentamycin, netilmycin, tobramycin. Macrolides such as amphotericin B, novobiocin, bacitracin, nystatin, clindamycin, polymyxins, colistin, rovamycin, erythromycin, spectinomycin, lincomycin, vancomycin Tetracyclines such as chlortetracycline, oxytetracycline, demeclocycline, rolitetracycline, doxycycline, tetracycline and minocycline. Other antibiotics such as chloramphenicol, rifamycin, rifampicin and thiamphenicol.

Chemotherapeutic agents such as the sulfonamides sulfadiazine, sulfamethizol, sulfadimethoxin, sulfamethoxazole, sulfadimidin, sulfamethoxypyridazine, sulfafurazole, sulfaphenazol, sulfalene, sulfisomidin, sulfamerazine, sulfisoxazole and trimethoprim with sulfamethoxazole or sulfametrole.

Urinary tract antiseptics such as methanamine, quinolones(norfloxacin, cinoxacin), nalidixic acid, nitro-compounds (nitrofurantoine, nifurtoinol) and oxolinic acid.

Drug for anaerobic infections such as metronidazole.

Drugs for tuberculosis such as aminosalicyclic acid, isoniazide, cycloserine, rifampicine, ethambutol, tiocarlide, ethionamide and viomycin.

Drugs for leprosy such as amithiozone, rifampicine, clofazimine, sodium sulfoxone and diaminodiphenylsulfone (DDS, dapsone).

Antifungal agents such as amphotericin B, ketoconazole, clotrimazole, miconazole, econazole, natamycin, flucytosine, nystatine and griseofulvin.
Antiviral agents such as aciclovir, idoxuridine, amantidine, methisazone, cytarabine, vidarabine and ganciclovir.
Chemotherapy of amebiasis such as chloroquine, iodoquinol, clioquinol, metronidazole, dehydroemetine, paromomycin, diloxanide, furoatetinidazole and emetine.
Anti-malarial agents such as chloroquine, pyrimethamine, hydroxychloroquine, quinine, mefloquine, sulfadoxine/pyrimethamine, pentamidine, sodium suramin, primaquine, trimethoprim and proguanil.
Anti-helminthiasis agents such as antimony potassium tartrate, niridazole, antimony sodium dimercaptosuccinate, oxamniquine, bephenium, piperazine, dichlorophen, praziquantel, diethylcarbamazine, pyrantel parmoate, hycanthone, pyrivium pamoate, levamisole, stibophen, mebendazole, tetramisole, metrifonate, thiobendazole and niclosamide.
Anti-inflammatory agents such as acetylsalicyclic acid, mefenamic acid, aclofenac, naproxen, azopropanone, niflumic acid, benzydamine, oxyphenbutazone, diclofenac, piroxicam, fenoprofen, pirprofen, flurbiprofen, sodium salicyclate, ibuprofensulindac, indomethacin, tiaprofenic acid, ketoprofen and tolmetin.
Anti-gout agents such as colchicine and allopurinol.
Centrally acting (opoid) analgesics such as alfentanil, methadone, bezitramide, morphine, buprenorfine, nicomorphine, butorfanol, pentazocine, codeine, pethidine, dextromoramide, piritranide, dextropropoxyphene, sufentanil and fentanyl.
Local anesthetics such as articaine, mepivacaine, bupivacaine, prilocaine, etidocaine, procaine, lidocaine and tetracaine.
Drugs for Parkinson's disease such as amantidine, diphenhydramine, apomorphine, ethopropazine, benztropine mesylate, lergotril, biperiden, levodopa, bromocriptine, lisuride, carbidopa, metixen, chlorphenoxamine, orphenadrine, cycrimine, procyclidine, dexetimide and trihexyphenidyl.
Centrally active muscle relaxants such as baclofen, carisoprodol, chlormezanone, chlorzoxazone, cyclobenzaprine, dantrolene, diazepam, febarbamate, mefenoxalone, mephenesin, metoxalone, methocarbamol and tolperisone.
Corticosteroids comprising:

Mineralocorticosteroids such as cortisol, desoxycorticosterone and flurohydrocortisone.
Glucocorticosteroids such as beclomethasone, betamethasone, cortisone, dexamethasone, fluocinolone, fluocinonide, fluocortolone, fluorometholone, fluprednisolone, flurandrenolide, halcinonide, hydrocortisone, medrysone, methylprednisolone, paramethasone, prednisolone, prednisone and triamcinolone (acetonide).
Androgens comprising:

Androgenic steroids used in therapy such as danazole, fluoxymesterone, mesterolone, methyltestosterone, testosterone and salts thereof.
Anabolic steroids used in therapy such as calusterone, nandrolone and salts thereof, dromostanolone, oxandrolone, ethylestrenol, oxymetholone, methandriol, stanozolol methandrostenolone and testolactone.
Antiandrogens such as cyproterone acetate.
Estrogens comprising estrogenic steroids used in therapy such as diethylstilbestrol, estradiol, estriol, ethinylestradiol, mestranol and quinestrol.
Anti-estrogens such as chlorotrianisene, clomiphene, ethamoxytriphetol, nafoxidine and tamoxifen.
Progestins such as allylestrenol, desogestrel, dimethisterone, dydrogesterone, ethinylestrenol, ethisterone, ethynadiol diacetate, etynodiol, hydroxyprogesterone, levonorgestrel, lynestrenol, medroxyprogesterone, megestrol acetate, norethindrone, norethisterone, norethynodrel, norgestrel, and progesterone.
Thyroid drugs comprising:

Thyroid drugs used in therapy such as levothyronine and liothyronine
Anti-thyroid drugs used in therapy such as carbimazole, methimazole, methylthiouracil and propylthiouracil.

Apart from bioactive agents which are water soluble, other water-soluble compounds can be incorporated such as anti-oxidants, ions, chelating agents, dyes, imaging compounds.
Preferred therapeutic agents are in the area of cancer (e.g. antitumor) and cardiovascular disease.
Methods of preparing lipophilic drug derivatives which are suitable for nanoparticle or liposome formulation are known in the art (see e.g., US 5,534,499 describing covalent attachment of therapeutic agents to a fatty acid chain of a

phospholipid). Drugs in the present invention can also be prodrugs.

The drug may be present in the inner, the outer, or both of the compartments of the carrier, e.g. in the cavity and/or in the shell of a liposome. The distribution of the drug is independent of the distribution of any other agents comprised in the drug carrier, such as a paramagnetic chemical shift reagent or a paramagnetic agent. A combination of drugs may be used and any of these drugs may be present in the inner, the outer, or both of the compartments of the drug carrier, e.g. in the cavity and/or in the shell of a liposome.

Thermosensitive carriers

The invention preferably provides for carriers that are thermosensitive. This means that the physical or chemical state of the carrier is dependent on its temperature.

Any thermosensitive carrier that can package a molecule of interest and that is intact at body temperature (i.e. 37°C) but destroyed at any other, non-body temperature that can be tolerated by a subject may be used. Carriers of the invention include but are not limited to thermosensitive micro- and nanoparticles, thermosensitive polymersomes, thermosensitive liposomes, thermosensitive nanovesicles and thermosensitive nanospheres.

Thermosensitive nanovesicles generally have a diameter of up to 100 nm. In the context of this invention, vesicles larger than 100 nm, typically up to 5000 nm, are considered as microvesicles. The word vesicle describes any type of micro- or nanovesicle. Vesicles, such as liposomal vesicles, typically include a cavity which may contain any substance of interest. In the invention this is preferred, as outlined above.

Thermosensitive nanospheres include but are not limited to spheres which are no smaller than 5 nanometers. Nanospheres typically do not contain a cavity, i.e. in this embodiment of the invention the CEST effect should be realized purely by chemically shifted protons of the paramagnetic chemical shift agent itself, that is comprised in the nanosphere.

Thermosensitive polymersomes include but are not limited to any polymer vesicle, including microvesicles and nanovesicles.

Thermosensitive liposomes include but are not limited to any liposome, including those having a prolonged half-life, e.g. pegylated, liposomes.

In order to make use of an optimal CEST contrast enhancement, it is preferred to employ thermosensitive carriers that have a semipermeable shell enclosing a cavity, such as liposomes. The advantage hereof is that the CEST contrast enhancement can be conducted on the basis of a paramagnetic chemical shift agent contained in the cavity, in interaction with a pool of protons or other MRI analytes also present in the cavity.

The term "semipermeable" is well understood in the art. In general it refers to the property of a shell, such as a membrane, to be selectively permeable, sometimes also denoted partially or differentially permeable. It indicates a structure that basically is closed in the sense that it is a not fully open wall, and preferably a mostly closed wall, (in this case a shell enclosing a cavity), that allows certain molecules or ions to pass through it by diffusion.

In this description, the semipermeability of the shell generally refers to its ability to allow the MR analyte to pass through it by diffusion. Hence, if the combination of analyte (such as water, or other small molecules comprising protons) and shell (such as a lipid bilayer) is such that the analyte is capable of passing through the shell by diffusion, the shell is considered semipermeable.

References on thermosensitive carriers having a semipermeable shell, are e.g. US 6,726,925, US 2006/0057192, US 2007/0077230A1 and JP 2006 - 306794. Based on the description of this invention, the reference to these disclosures will enable the person skilled in the art to execute CEST contrast enhancement using thermosensitive liposomes.

Liposomes are generally spherical vesicles comprising a bilayer membrane enclosing a cavity, or lumen. The bilayer can be made up of at least one phospholipid and may or may not comprise cholesterol. Liposomes can be composed of naturally-derived phospholipids with mixed lipid chains (like egg phosphatidylethanolamine), or of pure surfactant components like dioleoylphosphatidylethanolamine (DOPE). The term liposomes, as used in the description of the invention, includes lipid spheres usually denoted micelles.

A typical example of a semipermeable shell is also found in semipermeable membranes comprising a phospholipid bilayer. A phospholipid bilayer is the most permeable to small, uncharged solutes. Liposomes can be made on the basis of a phospholipid bilayer.

Thermosensitive liposomes for use in the invention ideally retain their structure at about 37°, i.e. human body temperature, but are destroyed at a higher temperature, preferably only slightly elevated above human body temperature, and preferably also above pyrexic body temperature. Typically about 42°C is a highly useful temperature for thermally guided drug delivery.

The required heat to raise the temperature of the thermosensitive drug carriers so as to promote the destruction of the thermosensitive carriers may be used. Heat can be applied in any physiologically acceptable way, preferably by using a focused energy source capable of inducing highly localized hyperthermia. The energy can be provided through, e.g., microwaves, ultrasound, magnetic induction, infrared or light energy.

Thermosensitive liposomes are known in the art. Liposomes according to the present invention may be prepared by any of a variety of techniques that are known in the art. See, e.g., U.S. Pat. No. 4,235,871; Published PCT applications WO 96/14057; New RRC, Liposomes: A practical approach, IRL Press, Oxford (1990), pages 33-104; Lasic,D.D., Liposomes from physics to applications, Elsevier Science Publishers, Amsterdam, 1993; Liposomes, Marcel Dekker, Inc., New York (1983).

Entrapment of a drug or other bio-active agent within liposomes of the present invention may also be carried out using any conventional method in the art. In preparing liposome compositions of the present invention, stabilizers such as antioxidants and other additives may be used as long as they do not interfere with the purpose of the invention. Examples include co-polymers ofN-isopropylacrylamide (Bioconjug. Chem. 10:412-8 (1999)).

Liposomes and other potential carriers based on a semipermeable shell enclosing a cavity, will generally be spherical. For use in the invention, it is preferred to render such spherical carriers aspherical. E.g. in the case of liposomes, this is done by subjecting the liposomes to a dialysis process against a hypertonic buffer solution, hence a buffer solution with a higher osmolarity compared to the solution at the inside of the liposomes. The dialysis causes a net diffusion of water from the inside of the liposomes to the bulk solution. This reduces the total inner volume of the liposomes. Since the surface area of the liposomes remains constant, the volume reduction forces the liposomes to deform and to assume an aspherical shape, such as a disk shape, a cigar shape, or any other aspherical shape.

<u>The paramagnetic chemical shift reagent</u>

In the invention, a paramagnetic shift reagent can be comprised in any manner in or on the carrier. It is preferred to have the shift reagent in sufficient interaction with a pool of protons by comprising both the reagent and the pool in the cavity of the carrier.

The paramagnetic chemical shift reagent or reagents can basically be any paramagnetic agent suitable to render the relatively large number of water molecules of a solution or dispersion in which it is contained, into a pool of protons that are chemically shifted regarding their MR resonance frequency, with respect to the surrounding protons of the bulk water molecules. As explained hereinbefore with respect to lipoCEST, the preferred thermosensitive drug carriers in which the paramagnetic chemical shift reagent and the internal pool of protons are contained, can be saturated through an RF pulse. As the preferred drug carriers comprise a shell, such as the lipid bilayer membrane of liposomes, that fundamentally allows exchange of protons with their direct environment, the saturation caused by the RF pulse will be transferred to the environment of the loaded, RF-saturated, thermosensitive drug carriers. Thus, upon conducting magnetic resonance imaging, the direct environment of the thermosensitive drug carriers will show a decreased signal intensity as compared to other bulk water molecules, and thus allows to detect the direct environment of the contrast agents due to a decreased signal intensity. The paramagnetic chemical shift reagent is to comprise a paramagnetic compound, *i.e.* any compound having paramagnetic properties. Preferably the paramagnetic compound comprises a paramagnetic metal, where the term metal refers to metallic nano- or microparticles, or metal ions, explicitly including metal ions complexed by chelate ligands. Paramagnetic metals are known to the skilled person, and do not require elucidation here. E.g., early and late transition metals, explicitly including chromium, manganese, iron, as well as lanthanides, such as gadolinium, europium, dysprosium, holmium, erbium, thulium, ytterbium.

The paramagnetic chemical shift reagent is to comprise a chelating structure capable of strongly binding to the paramagnetic metal and allowing the metal to interact with water, or with another suitable source of protons. With respect to suitable chelating structures, reference is made to P. Caravan et al., Chem. Rev., 99, 2293-2352 (1999). Preferably the water is at least transiently coordinated to the metal of the paramagnetic reagent. With respect to chemical shift mechanisms, reference is made to J. A. Peeters et al., Prog. Nucl. Magn. Reson. Spectr., 28, 283-350 (1999). In one embodiment, the chelating structure itself also comprises exchangeable protons, e.g. hydroxy, amine, or amide protons.

Suitably, the paramagnetic chemical shift reagent comprises a lanthanide ion coordinated with a chelating structure, e.g. macrocylic lanthanide(III) chelates derived from 1,4,7,10-tetraazacyclododecane-1,4,7,10-tetraacetic acid ($H_4$dota), 1,4,7,10-tetraazacyclododecane-α,α',α,"α"'-tetramethyl-1,4,7,10-tetraacetic acid ($H_4$dotma), and related ligands that allow for an axially coordinated water molecule in the paramagnetic reagent. In this respect reference is made to Aime et al., Angew. Chem. Int. Ed., 44, 5513-5515 (2005).

Preferably, the paramagnetic chemical shift reagent is water-soluble. Suitable chemical shift reagents are known to the person skilled in the art. The CEST contrast agents do not require any specific chemical shift reagent, as long as the shift reagent and the pool of protons have a sufficient interaction to result in a pool of chemically shifted protons.

Preferably, the paramagnetic shift reagent is a metal complex comprising a metal ion and a ligand that is based on a multidentate chelate ligand. More preferably, the interaction of the chemical shift reagent with the pool of protons is provided in the form of coordination. Thus it is preferred for the metal complex to have at least one coordination site of the metal left open for the coordination of at least one water molecule.

Examples of suitable water-soluble chemical shift reagents are [Ln(hpdo3a)($H_2O$)] (1), [Ln(dota)($H_2O$)]$^-$ (2), [Ln(dotma)($H_2O$)]$^-$ (3), [Ln(dotam)($H_2O$)]$^{3+}$ (4), and [Ln(dtpa)($H_2O$)]$^{2-}$ (5), including derivatives thereof and related compounds, with Ln being a lanthanide ion.

Preferably the paramagnetic chemical shift reagent is a lanthanide complex such as in formulae 1-5 below:

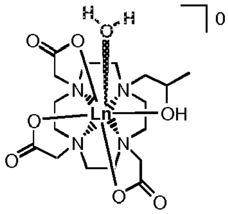

$[Ln(hpdo3a)(H_2O)]$

**1**

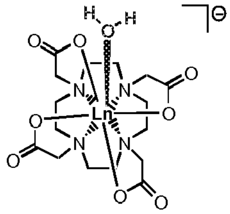

$[Ln(dota)(H_2O)]^-$

**2**

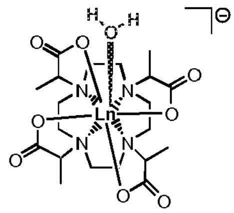

$[Ln(dotma)(H_2O)]^-$

**3**

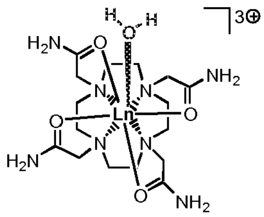

$[Ln(dotam)(H_2O)]^{3+}$

**4**

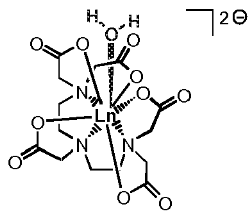

$[Ln(dtpa)(H_2O)]^{2-}$

**5**

wherein the lanthanide is $Eu^{3+}$, $Dy^{3+}$, $Ho^{3+}$, $Er^{3+}$, $Tm^{3+}$, $Yb^{3+}$ and preferably is $Tm^{3+}$ or $Dy^{3+}$.

The paramagnetic chemical shift reagent is typically comprised in the agent in an amount of from 1 mM to 2000 mM, preferably of from 10 mM to 1000 mM, and more preferably of from 50 mM to 200 mM.

Further contrast enhancement agents

The thermosensitive drug carriers of the invention may comprise a $T_1$ or $T_2$ reduction agent. In this respect reference is made to Aime et al., JACS, 129, 2430-2431 (2007). In this way an all-in-one concept is realized of $T_1$, $T_2$, and CEST contrast.

The chemical shift difference to the saturation transfer protons in the environment of the thermosensitive drug carriers, can be further enhanced by providing the thermosensitive drug carrier's membrane with a further paramagnetic agent, which is not necessarily a chemical shift reagent. Thus, the orientation of the aspherical carrier in the magnetic field is affected and the aforementioned bulk susceptibility effect is enhanced. The further paramagnetic agent is preferably an amphiphilic compound comprising a lanthanide complex (on the more polar side of the amphilic compound), and having an apolar tail which has a tendency to preferably integrate in and align with the lipid bilayer at the thermosensitive drug carrier's surface based on hydrophobic molecular interactions.

These amphilic paramagnetic complexes can e.g. be:

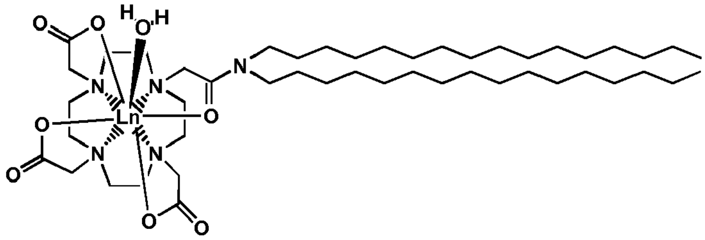

6

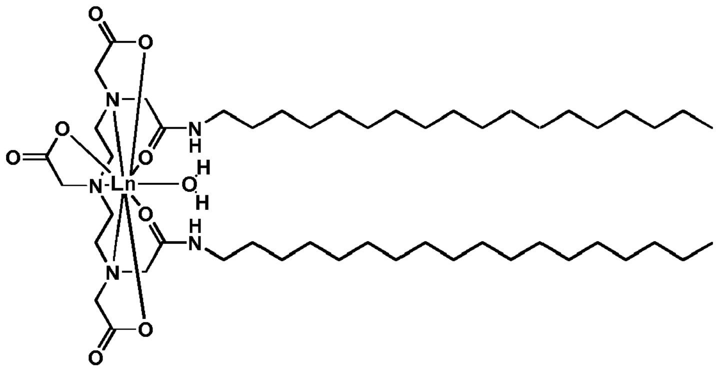

7

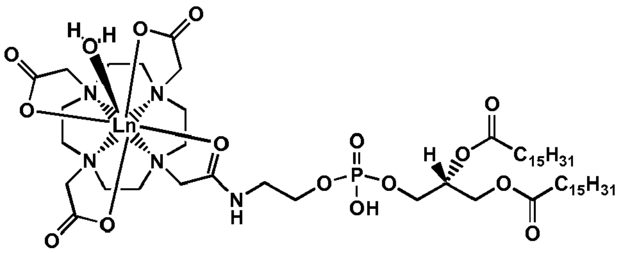

8

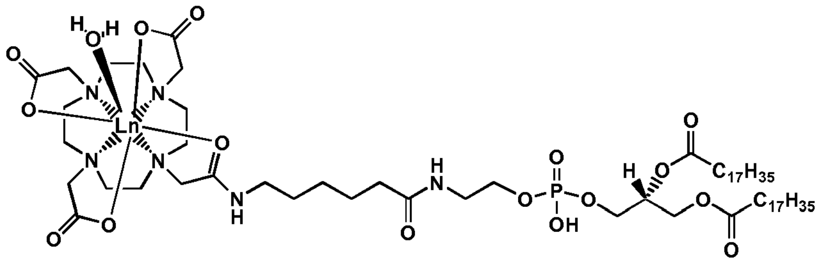

9

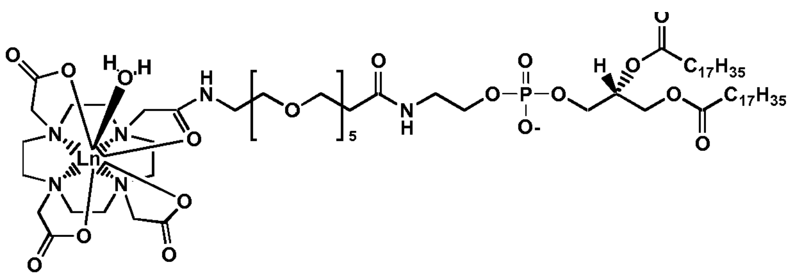

10

The paramagnetic chemical shift reagent loaded thermosensitive drug carriers can advantageously be combined with paramagnetic agents in or at its outer surface, e.g. the liposome shell. Such agents generally comprise an amphiphilic compound bearing a paramagnetic group, preferably a lanthanide complex (on the more polar side of the amphilic compound), and having an apolar tail which has a tendency to preferably integrate in and align with the lipid bilayer at the preferred thermosensitive liposomes' surface based on hydrophobic molecular interactions.

The lanthanide ion in the optional membrane-associated paramagnetic agent may be identical with or different from the lanthanide within the cavity of the contrast agent.

As is provided according to the invention, the paramagnetic chemical shift reagent can be encapsulated in thermosensitive drug carriers. In this way a pool of water protons is created that has a different chemical shift compared to that of the bulk water surrounding the carriers. The magnetic resonance of these chemically-shifted water protons can be saturated with an RF pulse of a sufficiently narrow band width. Since the water molecules at the inside of the contrast agent are exchanging quickly with the bulk water molecules surrounding the contrast agents, this saturation is transferred to the bulk water.

Hence, when used in practice, at the location of a CEST contrast agent based on thermosensitive drug carriers, the surrounding water (i.e. body fluid in the preferred use *in vivo*) will be visible as hypointense areas in the CEST-enhanced MR images. With CEST-enhanced MRI, we mean conventional MRI wherein, prior to excitation, the exchangeable-water resonance has been selectively saturated. The RF pulse used for saturation typically has a band-width of several Hertz to several hundred Hertz. The appropriate frequency for the pulse is usually known a priori from phantom or preclinical CEST-MRI studies, but can also be optimized during the actual clinical MRI examination.

Thus, the carriers of the present invention are detectable through MRI at any point in time before the carrier is opened-up. If they additionally comprise $T_1$ or $T_2$ contrast agents, also the drug release step upon opening-up of the carrier can be detected (the CEST contrast enhancement will work as long as the shell is closed and exchange of saturated protons can occur through diffusion, the $T_1$ or $T_2$ contrast enhancement will display its action when these contrast agents are enabled to interact with bulk water (i.e. body fluid, when said agents are released through the opening up of the shell).

The CEST contrast agents according to the invention can be used in a variety of ways. They can be applied to generate a desired level of MRI contrast in any aqueous environment. Its main use, which is also where the benefits of using thermosensitive drug carriers are enjoyed most, is to generate a local MRI contrast upon *in vivo* application. This can be by introducing the contrast agents, e.g. by injection into the blood or another body fluid of a living being, preferably a human being, and to perform a CEST contrast-enhanced MRI scan of the body, in whole or in part, of said being. The CEST contrast enhancement of bulk water molecules generated, allows the visibility of spots, such as tumors, where the regular body fluid presence is disturbed. Also, the contrast agents of the invention, in their lipid shell can be provided with disease-specific molecular probes, e.g. by having compounds possessing hydrophobic tail suitable to penetrate into the surface of the carrier (e.g. in the case of a phospholipid surface), wherein the other end of the compounds contains a ligand as desired.

This allows the contrast agents to preferentially locate at desired or suspect body sites which then can be made visible by MRI. This adds to the suitability of the drug carriers of the invention for localized delivery.

The CEST contrast agents of the invention preferably act on the basis of a pool of protons within the carrier, which exchange with fluid outside of the carrier. This exchange can be done by water-proton transfer, but also by proton transfer from other molecules small enough to pass the shell of the carrier, i.e. as preferred the lipid bilayer of thermosensitive liposomes.

It is to be understood that the invention is not limited to the embodiments and formulae as described hereinbefore. It is also to be understood that in the claims the word "comprising" does not exclude other elements or steps. Where an indefinite or definite article is used when referring to a singular noun e.g. "a" or "an", "the", this includes a plural of that noun unless something else is specifically stated.

The invention will be illustrated with reference to the following, non-limiting Example and the accompanying non-limiting Figure.

EXAMPLE

Liposomes with an average diameter of 100 nm were formed by the lipid film hydration technique coupled with sequential extrusion. MPPC (myristoyl palmitoyl glycero phosphocholine) and DPPC (dipalmitoyl glycero phosphocholine) were dissolved in a solution of $CHCl_3$/EtOH (4:1 v/v). The solvent was gently removed under reduced pressure and a thin lipidic film was obtained. The lipidic film was hydrated in a solution of 5 mM carboxyfluorescein and 65 mM [Tm(hpdo3a)($H_2O$)] in 20 mM HEPES (4-(2-hydroxyethyl)-1-piperazine ethane sulfonic acid) buffer at a pH of 7.4. The dispersion was extruded several times through polycarbonate membrane filters with pore diameters of 100 nm. The obtained liposomes were dialysed overnight at 4 °C to remove carboxyfluorescein and
[Tm(hpdo3a)($H_2O$)] that were not entrapped after hydration of the lipidic film. Dialysis was performed against a buffer with a high ionic strength (20 mM HEPES buffer containing 0.3 M NaCl) to obtain aspherical liposomes.

BRIEF DESCRIPTION OF THE DRAWING

Fig. 1 depicts a thermo-sensitive liposome for image-guided delivery of drug molecules. The schematic representation shows the release of a drug and a chemical shift reagent upon the application of an external trigger (e.g. heating, ultrasound or RF). In this case the MR image contrast is enhanced based on a lipoCEST mechanism.

## Claims

1. A drug carrier adapted for localized drug delivery, comprising a Chemical Exchange-dependent Saturation Transfer (CEST) contrast agent for Magnetic Resonance Imaging (MRI).

2. A drug carrier according to claim 1, comprising a thermosensitive material capable of affecting drug release.

3. A drug carrier according to claim 2, wherein the thermosensitive material is a semipermeable shell enclosing a cavity, wherein the cavity comprises a paramagnetic chemical shift reagent and a pool of proton analytes, and wherein the shell allows diffusion of the proton analytes.

4. A drug carrier according to claim 3, having a non-spherical shape.

5. A drug carrier according to claim 3 or 4 , wherein the pool of proton analytes comprises water.

6. A drug carrier according to any one of the preceding claims, being a thermosensitive nanoparticle selected from the group consisting of liposomes, polymersomes, nanocapsules, and mixtures thereof.

7. A drug carrier according to any one of the preceding claims comprising a metal complex having a metal ion and a ligand that is based on a multidentate chelate ligand, as a paramagnetic chemical shift reagent.

8. A drug carrier according to claim 7, wherein the metal complex has at least one coordination site of the metal left open for the coordination of at least one water molecule.

9. A drug carrier according to claim 8, wherein the paramagnetic shift reagent is selected from the group consisting of $[Ln(hpdo3a)(H_2O)]$, $[Ln(dota)(H_2O)]^-$, $[Ln(dotma)(H_2O)]^-$, $[Ln(dotam)(H_2O)]^{3+}$, $[Ln(dtpa)(H_2O)]^{2-}$, derivatives thereof, and mixtures thereof, with Ln being a lanthanide ion.

10. A drug carrier according to any one of the preceding claims comprising a paramagnetic agent in or at its outer surface.

11. A drug carrier according to claim 10, wherein the paramagnetic agent is selected from the group consisting of at least one of the lanthanide ions $Dy^{3+}$, $Ho^{3+}$, $Er^{3+}$, $Tm^{3+}$, or $Yb^{3+}$ complexed by a multidentate chelating molecule bearing at least one hydrophobic group that comprises at least 6 carbon atoms.

12. A drug carrier according to any one of the preceding claims, comprising a drug for the treatment of cancer or cardiovascular disorders.

13. A method for the MRI guided delivery of a bio-active agent to a subject, comprising the administration of a drug carrier according to any one of the preceding claims to said subject, rendering an MR image using the CEST contrast enhancement provided by said administered drug carrier, and allowing the drug carrier to release the bio-active agent.

14. The use of a CEST MRI contrast agent as a drug carrier.

15. A use according to claim 14, wherein the CEST MRI contrast agent is a thermosensitive liposome comprising in its lumen a paramagnetic chemical shift reagent and a pool of proton analytes.

16. A use according to claim 14 or 15, wherein the CEST MRI contrast agent is a non-spherical thermosensitive liposome the shell of which comprises a paramagnetic agent.

**17.** A use according to claim 16, wherein the liposome does not comprise a paramagnetic shift reagent substantially interacting with the analytes.

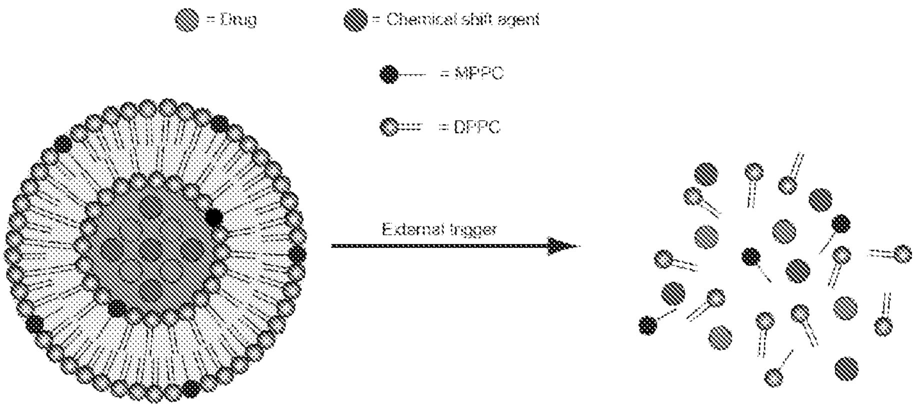


FIG. 1

European Patent Office

# EUROPEAN SEARCH REPORT

Application Number
EP 07 12 2621

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| D,X | TERRENO E ET AL: "From spherical to osmotically shrunken paramagnetic liposomes: an improved generation of LIPOCEST MRI agents with highly shifted water protons" ANGEWANDTE CHEMIE. INTERNATIONAL EDITION, WILEY VCH VERLAG, WEINHEIM, DE, vol. 46, no. 6, 29 January 2007 (2007-01-29), pages 966-968, XP002470079 ISSN: 1433-7851 * page 967, column 1, paragraph 2 - column 2, paragraph 2 * * compound 1 * ----- | 1-17 | INV. A61K41/00 A61K49/00 A61K49/18 A61K9/00 A61K9/127 |
| X | TERRENO E. ET AL.: "Highly shifted LIPOCEST agents based on the encapsulation of neutral polynuclear paramagnetic shift reagents" CHEMICAL COMMUNICATION, vol. 8, no. 5, 26 November 2007 (2007-11-26), pages 600-602, XP002476682 * page 601, column 2, paragraph 4 * * compounds 1-3 * ----- | 1-17 | TECHNICAL FIELDS SEARCHED (IPC): A61K |
| D,X | AIME S ET AL: "Gd-loaded liposomes as T1, suseptibility, and CEST agents, all in one" JOURNAL OF THE AMERICAN CHEMICAL SOCIETY, AMERICAN CHEMICAL SOCIETY, WASHINGTON, DC, US, vol. 129, no. 9, 7 March 2007 (2007-03-07), pages 2430-2431, XP002470078 ISSN: 0002-7863 * page 2430, column 1, paragraph 3 - column 2, paragraph 1 * ----- | 1-9, 12-15 | |

-/--

The present search report has been drawn up for all claims

| Place of search | Date of completion of the search | Examiner |
|---|---|---|
| The Hague | 22 April 2008 | Monami, Amélie |

2

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

European Patent Office

# EUROPEAN SEARCH REPORT

Application Number
EP 07 12 2621

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2006/032705 A (GUERBET SA [FR]; PORT MARC [FR]) 30 March 2006 (2006-03-30)<br>* examples 1-9 *<br>* page 11, line 17 - page 12, line 22 *<br>* page 13, line 19 - page 16, line 18 *<br>----- | 1-3,5-15 | |
| X | WO 2007/115115 A (KEREOS INC [US]; LANZA GREGORY M [US]; WICKLINE SAMUEL A [US]; KIEFER) 11 October 2007 (2007-10-11)<br>* page 2, paragraph 8 - paragraph 9 *<br>* page 11, paragraph 53 - paragraph 54 *<br>* page 13, paragraph 57 - page 17, paragraph 59 *<br>* examples 1-7 *<br>----- | 1,7-14 | |
| A | US 6 468 505 B1 (LANG PHILIPP [US] ET AL) 22 October 2002 (2002-10-22)<br>* column 2, line 15 - line 37 *<br>* column 3, line 11 - line 12 *<br>* column 3, line 39 - line 49 *<br>* column 5, line 15 - line 27 *<br>* examples 1-7 *<br>----- | | TECHNICAL FIELDS SEARCHED (IPC) |
| A | US 2004/101969 A1 (VIGLIANTI BENJAMIN L [US] ET AL) 27 May 2004 (2004-05-27)<br>* page 1, paragraph 3 *<br>* page 2, paragraph 20 *<br>* page 2, paragraph 11 *<br>----- | | |

The present search report has been drawn up for all claims

| Place of search | Date of completion of the search | Examiner |
|---|---|---|
| The Hague | 22 April 2008 | Monami, Amélie |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

2

EPO FORM 1503 03.82 (P04C01)

## ANNEX TO THE EUROPEAN SEARCH REPORT ON EUROPEAN PATENT APPLICATION NO.

EP 07 12 2621

This annex lists the patent family members relating to the patent documents cited in the above-mentioned European search report.
The members are as contained in the European Patent Office EDP file on
The European Patent Office is in no way liable for these particulars which are merely given for the purpose of information.

22-04-2008

| Patent document cited in search report | Publication date | Patent family member(s) | Publication date |
|---|---|---|---|
| WO 2006032705 A | 30-03-2006 | EP 1793868 A2<br>US 2007292354 A1 | 13-06-2007<br>20-12-2007 |
| WO 2007115115 A | 11-10-2007 | NONE | |
| US 6468505 B1 | 22-10-2002 | NONE | |
| US 2004101969 A1 | 27-05-2004 | US 2007197904 A1 | 23-08-2007 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5534499 A
- US 6726925 B
- US 20060057192 A
- US 20070077230 A1
- JP 2006306794 A
- US 4235871 A
- WO 9614057 A


### Non-patent literature cited in the description


- **PONCE et al.** *J Natl Cancer Inst,* 2007, vol. 99, 53-63
- **TERRENO,E. et al.** *Angew. Chem. Int. Ed.,* 2007, vol. 46, 966-968
- Liposomes: A practical approach. New RRC. IRL Press, 1990, 33-104
- **LASIC,D.D.** Liposomes from physics to applications. Elsevier Science Publishers, 1993
- Liposomes. Marcel Dekker, Inc, 1983
- *Bioconjug. Chem.,* 1999, vol. 10, 412-8
- **P. CARAVAN et al.** *Chem. Rev.,* 1999, vol. 99, 2293-2352
- **J. A. PEETERS et al.** *Prog. Nucl. Magn. Reson. Spectr.,* 1999, vol. 28, 283-350
- **AIME et al.** *Angew. Chem. Int. Ed.,* 2005, vol. 44, 5513-5515
- **AIME et al.** *JACS,* 2007, vol. 129, 2430-2431